# EUROPÄISCHE PATENTSCHRIFT

(11) **EP 2 462 109 B1**
(45) Veröffentlichungstag und Bekanntmachung des Hinweises auf die Patenterteilung: **19.03.2014**
(21) Anmeldenummer: 10739359.7
(22) Anmeldetag: 02.08.2010
(51) Int. Cl.: C07C 263/04, C07C 263/06, C07C 265/14

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 04.08.2009 EP 09167199
(43) Veröffentlichungstag der Anmeldung: 13.06.2012
(73) Patentinhaber: BASF SE, 67056 Ludwigshafen (DE)
(72) Erfinder: GEIßLER, Bernhard, 67281 Kirchheim (DE); STROEFER, Eckhard, 68163 Mannheim (DE); SCHMIDT, Andreas, 01987 Schwarzheide (DE); KLÖTZER, Matthias, 01945 Kroppen (DE)
(86) Internationale Anmeldenummer: PCT/EP2010/061177
(87) Internationale Veröffentlichungsnummer: WO 2011/015541

(56) Entgegenhaltungen:
- EP-A2- 0 480 493
- WO-A1-2008/025659
- US-B2- 7 329 776
- J. NATANSON: "Uber zwei neue künstliche Bildungsweisen des Harnstoffs", JUSTUS LIEBIGS ANNALEN DER CHEMIE, Bd. 98, Nr. 3, 1856, Seiten 287-291, XP002613759, VERLAG CHEMIE GMBH. ISSN: 0075-4617

## Beschreibung

Gegenstand der Erfindung ist ein verbessertes mehrstufiges Verfahren zur kontinuierlichen Herstellung von Diisocyanaten durch Umsetzung der korrespondierenden Diamine mit Kohlensäurederivaten und Alkoholen in niedermolekulare monomere Urethane und deren thermische Spaltung.

Die technischen Verfahren zur Herstellung von organischen Polyisocyanaten, wie z.B. von aromatischen, aliphatischen oder cycloaliphatischen Polyisocyanaten, beruhen auf der Phosgenierung der entsprechenden organischen Polyamine zu Polycarbamidsäurechloriden und deren thermische Spaltung zu den Polyisocyanaten und Chlorwasserstoff. Abgesehen von den schwerwiegenden Umweltschutz-, Entsorgungs- und Sicherheitsproblemen, die der Einsatz von Phosgen mit sich bringt, sind diese Verfahren mit weiteren entscheidenden Nachteilen behaftet. So gelingt die Herstellung von aliphatischen oder cycloaliphatischen Polyisocyanaten aufgrund der stärkeren Basizität der Ausgangspolyamine nur mit recht mäßigen Raum-Zeit-Ausbeuten. Nachteilig ist ferner die Bildung von unerwünschten Nebenprodukten, die, bereits in Spuren vorliegend, zu starken Verfärbungen der Polyisocyanate führen können. Bei der Hexamethylendiisocyanat-1,6 (HDI)-Herstellung entstehen z.B. mehrere Nebenprodukte, von denen das wichtigste, 6-Chlorhexylisocyanat, zudem den Nachteil besitzt, daß es nur mit erheblichem destillativem Aufwand vom HDI abgetrennt werden kann.

Problematisch bei dieser Verfahrensweise sind insbesondere der hohe Umsatz von Chlor über Phosgen und Carbamidsäurechlorid in Chlorwasserstoff, die Toxizität des Phosgens sowie die Korrosivität des Reaktionsgemisches, die Labilität der in der Regel eingesetzten Lösungsmittel und die Bildung halogenhaltiger Rückstände.

Die thermische Spaltung von (cyclo)aliphatischen und insbesondere aromatischen Mono- und Diurethanen in die entsprechenden Isocyanate und Alkohol ist seit langem bekannt und kann sowohl in der Gasphase bei hohen Temperaturen als auch in der flüssigen Phase bei vergleichsweise niedrigen Temperaturen ausgeführt werden.

Bei der Umsetzung von Diaminen mit Harnstoff und Alkohol bilden sich neben den gewünschten spaltbaren Diurethanen als Nebenprodukte Carbonate sowie häufig auch Carbaminsäureester.

EP 566925 A2, EP 1512680 A, EP 1512681 A, EP 1512682 A und EP 1602643 A1 beschreiben die Herstellung von Diisocyanaten durch Umsetzung der korrespondierenden Diamine, Harnstoff und Alkohol optional in Gegenwart von Carbonaten und/oder Carbaminsäureestern. Diese Carbonate und Carbaminsäureester werden destillativ aus dem Reaktionsgemisch abgetrennt und können optional in die Reaktion rückgeführt werden. Eine spezielle Nutzung oder Behandlung der abgetrennten Carbonate außer der destillativen Aufreinigung erfolgt nicht. Nachteilig ist ferner, daß eine einfache Rückführung der Carbonate in die Reaktion zu einer Aufpegelung im kontinuierlichen Betrieb führt. Da Carbonate starke Alkylierungsmittel sind, führt deren Vorliegen in der Reaktion zur Alkylierung des eingesetzten Diamins, das somit nach einer Alkylierung der Isocyanatbildung nicht mehr zur Verfügung steht, sondern als Nebenprodukt im Reaktionsgemisch verbleibt.

Nachteilig an diesen Verfahren ist, daß das bei der Reaktion anfallende Carbonat durch einfache Rückführung in die Reaktionsstufe nicht oder nur unvollständig verwertet werden kann. Als Möglichkeit der Verwertung für das abgetrennte Carbonat bleibt dessen Verwertung als Reinstoff außerhalb des Verfahren oder die thermische Verwertung, d.h. Verbrennung und Nutzung der Abwärme.

Die Aufgabe der vorliegenden Erfindung bestand darin, ein Verfahren zur Herstellung von Diisocyanaten aus Diaminen, Harnstoff und Alkohol zur Verfügung zu stellen, in dem das bei der Reaktion anfallende Carbonat innerhalb des Verfahrens verwendet werden kann.

Diese Aufgabe konnte gelöst werden durch ein Verfahren zur Herstellung von Diisocyanaten durch Umsetzung der korrespondierenden Diamine mit Harnstoff und mindestens einem Alkohol zum korrespondierenden Urethan (Urethanisierung) unter Bildung des Carbonats des eingesetzten Alkohols und Spaltung des so erhaltenen Urethans zum Isocyanat (Urethanspaltung), in dem man zumindest einen Teil des gebildeten Carbonats aus dem Reaktionsgemisch abtrennt, außerhalb der Urethanisierung mit Ammoniak umsetzt und das so gebildete Reaktionsgemisch in die Urethanisierung führt.

Gegenstand der Erfindung ist ferner ein mehrstufiges Verfahren zur kontinuierlichen Herstellung von Diisocyanaten durch Umsetzung des korrespondierenden Diamins mit Harnstoff und mindestens einem Alkohol in die entsprechenden Urethane unter Bildung des Carbonats des eingesetzten Alkohols in mindestens einem Reaktor und thermische Spaltung der Urethane, das folgende Stufen umfaßt und in dem man
a) Diamin mit Harnstoff in Gegenwart mindestens eines chloridfreien Katalysators oder vorzugsweise in Abwesenheit von Katalysatoren und in Abwesenheit oder vorzugsweise in Gegenwart mindestens eines Alkohols vermischt,
b) das aus a) erhaltene Gemisch in mindestens einem Verweilzeitreaktor zu dem korrespondierenden Urethan und Carbonat umsetzt
c) den dabei entstehenden Ammoniak abtrennt,
d) aus dem Austrag aus c) überschüssigen Alkohol, Carbonat und weitere leichtsiedende Nebenkomponenten abtrennt,
e) das vom Alkohol und leichtsiedenden Komponenten befreite Urethan aus (d) zumindest teilweise einer Destillation zuführt,
f) die Urethane im Destillat aus (e) und den gegebenenfalls nicht der Destillation (e) zugeführten Anteil aus (d) in einer kontinuierlichen Spalteinrichtung in das entsprechende Diisocyanat und Alkohol spaltet,
g) das aus (f) erhaltene Rohisocyanat in mindestens einer Destillation reinigt und anfallende Destillationsrückstände erneut der Spaltung (f) zuführt und/oder mit Alkohol zu Urethanen umwandelt und der Reaktionseinheit (b) zuführt,
h) man den Reaktionsaustrag aus (f), der einen hohen Anteil an Urethanen und verwertbaren Verbindungen enthält, durch Umsatz mit mindestens einem Alkohol erneut in Urethane überführt und dieses Reaktionsgemisch in die Stufe a) und/oder b) führt und
i) man das in d) abgetrennte Carbonat zumindest teilweise mit Ammoniak umsetzt und das Produkt dieser Umsetzung in die Stufe a) und/oder b) führt.

Das erfindungsgemäße Verfahren weist eine effektivere Nutzung des als Nebenprodukt entstehenden Carbonats auf, als im Stand der Technik bekannte Verfahren.

Rein formal betrachtet kann das erfindungsgemäße Verfahren schematisch durch folgende Gleichung bilanziert werden:

R-(NH₂)ₙ + n H₂N(CO)NH₂ + n R'OH → R(NCO)ₙ + n R'OH + 2n NH₃

Im Rahmen dieser Schrift wird mit "Carbonat" das organische Carbonat des eingesetzten Alkohols bezeichnet, das die Formel

R'-O-(CO)-O-R'

aufweist.

Als "Carbaminsäureester" wird der Ester der Carbaminsäure mit dem eingesetzten Alkohol bezeichnet, der die Formel

R'-O-(CO)-NH₂

aufweist.

Zur Herstellung der als Zwischenprodukte verwendbaren monomeren Urethane eignen sich Amine der Formel R(NH₂)ₙ, in der R einen mehrwertigen, vorzugsweise zweiwertigen organischen Rest, wie z.B. einen gegebenenfalls substituierten, beispielsweise mit einer Alkylgruppe substituierten aromatischen oder vorzugsweise einen linearen oder verzweigtkettigen, aliphatischen oder gegebenenfalls substituierten cycloaliphatischen Rest bedeutet.

Als geeignete aromatische Polyamine beispielhaft genannt seien 2,4- und 2,6-Toluylendiamin, 4,4'-, 2,4'- und 2,2'-Diamino-diphenylmethane und die entsprechenden Isomerengemische.

Als aliphatische oder cycloaliphatische Polyamine kommen beispielsweise in Betracht: Butandiamin-1,4, 2-Ethylbutandiamin-1,4, Octandiamin-1,8, Decandiamin-1,10, Dodecandiamin-1,12, Cyclohexandiamin-1,4, 2-Methyl-, 4-Methyl-cyclohexandiamin-1,3, 1,3- und 1,4-Diaminomethylcyclohexan, 4,4'-Di(aminocyclohexyl)methan und 3 (bzw. 4), 8 (bzw. 9)-Bis(aminomethyl)-tricyclo[5.2.1.0^{2.6}]decan-Isomerengemische. Vorzugsweise Verwendung finden 2-Methylpentandiamin-1,5, 2,2,4- bzw. 2,4,4-Trimethylhexandiamin-1,6, Dicyclohexylmethylendiamin (H12MDA) und insbesondere Hexandiamin-1,6 und 3-Aminomethyl-3,5,5-trimethylcyclohexylamin.

Als Alkohole eignen sich prinzipiell alle aliphatischen und cycloaliphatischen, bevorzugt aliphatischen Alkohole. Vorzugsweise wird man jedoch solche auswählen, deren Siedepunkte genügend weit vom Siedepunkt des durch die thermische Spaltung erhaltenen Diisocyanates entfernt liegen, so daß eine möglichst quantitative Trennung der Spaltprodukte Diisocyanat und Alkohol möglich ist.

Aus diesen Gründen finden daher vorzugsweise Alkohole, wie z.B. Methanol, Ethanol, n-Propanol, n-Butanol, iso-Butanol, n-Pentanol, iso-Pentanol, n-Hexanol, Isohexanole, Cyclopentanol, Cyclohexanol, 2-Ethylhexanol, Decanol oder Gemische der genannten Alkohole, bevorzugt Methanol, n-Butanol und/oder iso-Butanol, insbesondere aber n-Butanol und/oder iso-Butanol Verwendung.

Die einzelnen Stufen des Verfahrens werden im Folgenden beschrieben:

### a) Vermischung der Reaktionskomponenten

Die Vermischung der Eduktströme kann in beliebigen Apparaten erfolgen, die dem Fachmann an sich bekannt sind. Die Vermischung im Schritt (a) kann auch mit der Reaktion im Schritt (b) gemeinsam erfolgen. Bevorzugt kann die Vermischung in einer geeigneten gesonderten Mischeinrichtung erfolgen, besonders bevorzugt in einer speziellen Mischeinrichtung, die sich durch geringe Mischzeiten auszeichnet.

Gesonderte Mischeinrichtungen sind beispielsweise Mischkreise, Rührkessel, Rührkesselkaskaden, Rohre mit statischen Mischern oder Mischpumpen.

Es ist möglich, die Stufe (a) (Vermischung) und (b) (Urethanbildung) zu trennen oder zu vereinigen. Zumeist wird, je nach Reaktionsbedingungen, die Urethanbildung bereits bei der Vermischung der Edukte einsetzen.

Zur Herstellung der Urethane in der Reaktionsstufe (a) wird das Diamin mit Harnstoff und mindestens einem, bevorzugt genau einem Alkohol in einem molaren Verhältnis von Amin, Harnstoff und Alkohol wie 1 : 2 bis 20 : 5 bis 40 bei Temperaturen von 50 - 300 °C und insbesondere bei 180 - 220 °C unter einem Druck von 0,1 bis 30 bar, vorzugsweise 5 - 20 bar zur Reaktion gebracht. Bei diesen Reaktionsbedingungen ergeben sich für das erfindungsgemäße Verfahren mittlere Reaktionszeiten von Bruchteilen von Sekunden bis Minuten.

Um das signifikante Einsetzen der Urethanbildung bereits während der Vermischung der Komponenten zu verhindern bzw. zu verringern, ist es in der Regel ausreichend, die Komponente bei einer Temperatur unterhalb von 150°C zu vermischen.

Die Umsetzung in der Reaktionsstufe (a) kann in Gegenwart von Dialkylcarbonaten, zweckmäßigerweise in einer Menge von 0,1 bis 30 Mol%, vorzugsweise 1 bis 10 Mol% oder Carbamidsäurealkylestern zweckmäßigerweise in einer Menge von 1 bis 20 Mol%, vorzugsweise von 5 bis 15 Mol%, bezogen auf Diamin durchgeführt werden. Insbesondere verwendet werden dabei Mischungen aus Dialkylcarbonaten und Carbamidsäurealkylestern in den genannten Mengenverhältnissen, die aus der erfindungsgemäßen Stufe i) stammen. Als Dialkylcarbonate und/oder Carbamidsäureester setzt man bevorzugt solche ein, deren Alkylreste dem Alkylrest des verwendeten Alkohols entsprechen.

Wie bereits dargelegt, kann die Umsetzung in der Reaktionsstufe (a) auch in Gegenwart von Katalysatoren erfolgen. Diese werden zweckmäßigerweise in Mengen von 0,001 bis 20 Gew% vorzugsweise 0,001 bis 5 Gew% insbesondere 0,01 bis 0,1 Gew%, bezogen auf das Gewicht des Amins, eingesetzt.

Als Katalysatoren eignen sich anorganische oder organische Verbindungen, die ein oder mehrere Kationen, vorzugsweise ein Kation von Metallen der Gruppe IA, IB, IIA, IIB, IIIB, IVA, IVB, VA, VB, VIB, VIIB, VIIIB des Periodensystems der Elemente enthalten, definiert gemäß Handbook of Chemistry and Physics 14th Edition, publiziert von Chemical Rubber Publishing Co., 23 Superior Ave. N.E., Cleveland, Ohio. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen und Cobalt.

Der Katalysator kann weiterhin mindestens ein Anion enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate.

Wenn ein Katalysator eingesetzt wird, dann wird erfindungsgemäß ein solcher Katalysator eingesetzt, der keine erhöhte Korrosivität aufweist, besonders bevorzugt ein nicht chlorid-haltiger Katalysator, ganz besonders bevorzugt ein nicht halogenid-haltiger Katalysator, insbesondere wird kein Katalysator in die Reaktionsstufe dosiert.

Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminiumacetylacetonat, Aluminium-iso-butylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphtenat, Vanadium-(III)-chlorid, Vanadiumacetylacetonat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Als bevorzugte Katalysatoren seien beispielhaft folgende Verbindungen genannt: Lithiumbutanolat, Aluminiumacetylacetonat, Zinkacetylacetonat, Titantetrabutanolat und Zirkontetrabutylat.

Die Mischzeit in speziellen Mischeinrichtungen mit kurzer Mischzeit beträgt üblicherweise von 0,0001 s bis 2 s, bevorzugt von 0,0005 bis 1 s, besonders bevorzugt von 0,001 bis 0,5 s, ganz besonders bevorzugt von 0,005 bis 0,2 s und insbesondere von 0,007 bis 0,1 s. Als Mischzeit ist diejenige Zeit zu verstehen, die von dem Beginn des Mischvorgangs vergeht, bis 97,5 % der Fluidelemente des erhaltenen Gemisches einen Mischungsbruch haben, der bezogen auf den Wert des theoretischen Endwertes des Mischungsbruchs des erhaltenen Gemisches beim Erreichen des Zustandes perfekter Mischung weniger als 2,5 % von diesem Endwert des Mischungbruches abweichen (zum Konzept des Mischungsbruches siehe z.B. J.Warnatz, U.Maas, R.W. Dibble: Verbrennung, Springer Verlag, Berlin Heidelberg New York, 1997, 2. Auflage, S. 134.).

Als Mischeinrichtung bevorzugt wird ein Mischkreis, ein Rührbehälter, eine Mischpumpe oder eine Düsenmischeinrichtung, beispielsweise Koaxialmischdüsen, Y- oder T-Mischer, oder eine Vortex-Impinging-Jet-Mischkonfiguration eingesetzt, bevorzugt ein Mischkreis, ein Rührbehälter, eine Mischpumpe oder eine Düsenmischeinrichtung.

Bei der Verwendung eines Mischkreises oder eines Rührbehälters als Mischeinrichtung ist es wichtig, dass die Aminlösung mit hoher Geschwindigkeit eingedüst wird. Üblicherweise liegen die Geschwindigkeiten zwischen 10 und 100 m/s, bevorzugt zwischen 20 und 80 m/s.

Bevorzugt wird eine Mischdüse und eine Mischpumpe als Mischeinrichtung eingesetzt. Besonders bevorzugt wird als Mischeinrichtung eine Mischdüse verwendet. Hierbei ist es wichtig, dass sowohl der Alkohol- als auch der Amineduktstrom mit hoher Geschwindigkeit in die Mischdüse eingeleitet werden. Die Geschwindigkeiten betragen zwischen 10 und 100 m/s, bevorzugt zwischen 20 und 80 m/s.

Dabei liegt der Druck in den Zuleitungen zur Düse erheblich höher als im Ausgang der Mischdüse, üblicherweise jedoch nicht höher als 110 bar abs, bevorzugt nicht höher als 100 bar abs, besonders bevorzugt beträgt der Druck von 5 bis 95 bar abs, ganz besonders bevorzugt von 10 bis 50 bar abs und insbesondere von 10 bis 30 bar abs.

Der Druck am Ausgang der Mischeinrichtung liegt in der Regel oberhalb des Reaktionsdrucks in Stufe b), beispielsweise zwischen 5 und 100 bar, bevorzugt zwischen 10 und 80 bar, besonders bevorzugt zwischen 10 und 50 bar.

Die Temperatur des Austrages aus der Mischeinrichtung beträgt in der Regel zwischen 25 und 240 °C, bevorzugt 30 - 190 und besonders bevorzugt 40 - 180 °C.

Der Austrag aus der Mischeinrichtung kann vor Einleiten in die Stufe b) mit Hilfe eines Wärmetauschers auf die dort gewünschte Temperatur gebracht werden. Das Überführen des Reaktionsaustrages aus der Stufe a) in die nachfolgende Stufe kann vorteilhaft über Druckhalteventile erfolgen, wobei der Druck am Ausgang von Stufe a) um mindestens 1 bar, bevorzugt mindestens 2 bar, besonders bevorzugt mindestens 3 bar oberhalb des in Stufe b) herrschenden Druckes betragen sollte.

### b) Reaktion des Gemisches aus a)

Die die Mischeinrichtung verlassende Flüssigphase wird nun mindestens einem, bevorzugt genau einem zweiphasig (gasförmig/flüssig) betriebenen Reaktor zugeführt. Dabei kann es sich um einen nicht-rückvermischten, beispielsweise Rührkessel, oder bevorzugt um einen nicht- oder wenig rückvermischten Reaktor handeln, beispielsweise Rohrreaktoren oder Rührkesselkaskaden. Bevorzugt wird das Gemisch einem Rohrreaktor zugeführt oder mehreren Reaktoren, die von ihrer Verweilzeitverteilung einem Rohrreaktor ähneln, in dem die Gasphase mit der Flüssigphase im Gleichstrom geführt werden.

Der Rohrreaktor sollte bevorzugt weitestgehend rückvermischungsfrei sein. Dies wird beispielsweise erreicht durch das Verhältnis des Durchmessers des Rohrreaktors zu dessen Länge oder durch Einbauten, wie Lochböden, Schlitzböden oder statische Mischer. Bevorzugt wird die Rückvermischungsfreiheit durch das Verhältnis von Länge zu Durchmesser des Rohrreaktors erreicht.

Als Rohrreaktor eignen sich beispielsweise solche Rohre, deren Längen- zu Durchmesserverhältnis größer als 5 ist, bevorzugt größer als 6, besonders bevorzugt größer als 10.

Die Bodensteinzahl des Rohrreaktors sollte größer als 5 sein, bevorzugt größer als 6, besonders bevorzugt größer als 10, ganz besonders bevorzugt von 10 bis 600 und insbesondere von 10 bis 100.

Ein Aspekt, der wesentlich zur Erfindung beiträgt, ist das Vorliegen einer Strömung, die idealerweise eine Pfropfenströmung (Kolbenströmung, plug-flow) ist und dieser in der Realität so weit wie erforderlich angenähert werden soll. Dazu wird die axiale Durchmischung, also eine Durchmischung entlang der Flußrichtung durch den Reaktor, möglichst verringert und die Strömung ist idealerweise turbulent.

Dies wird in der Praxis erreicht durch hohe Strömungsgeschwindigkeiten und geringe Querschnittsflächen beispielsweise in Strömungsrohren.

Der Rohrreaktor kann eine beliebige Orientierung im Raum aufweisen. Bevorzugt wird er als senkrechter Rohrreaktor aufgebaut, der besonders bevorzugt von unten nach oben durchströmt wird.

Der Rohrreaktor kann isotherm oder bevorzugt temperiert ausgeführt werden. Eine Temperierung kann durch eine Mantelheizung oder durch innenliegende Rohre oder Platten erfolgen. Die Beheizung erfolgt bevorzugt durch den Mantel.

Selbstverständlich kann der Rohrreaktor auch aus mehreren seriell geschalteten Rohrstücken bestehen, solange die Rückvermischungsfreiheit gewährleistet bleibt. Falls erforderlich können optional im Verlauf des Rohrreaktors, beispielsweise zwischen derartige Rohrstücke Phasenscheider zur Trennung von flüssiger und gasförmiger Phase vorgesehen werden, in denen während der Reaktion entstandener Ammoniak abgetrennt werden kann, so daß das Gleichgewicht der Reaktion verschoben wird.

Zur Vergrößerung der Produktionskapazität können erfindungsgemäß auch mehrere Rohrreaktoren parallel geschalten werden.

Gegebenenfalls kann in den Rohrreaktor, wie oben ausgeführt, an einer oder mehreren Stellen, beispielsweise am Anfang und in der Mitte des Rohrreaktors, noch Harnstoff und/oder Alkohol oder bevorzugt Amin nachdosiert werden.

Die mittlere Verweilzeit im Rohrreaktor beträgt in der Regel 10 Sekunden bis 5 Stunden, bevorzugt 20 Sekunden bis 20 Minuten, besonders bevorzugt 30 Sekunden bis 10 Minuten.

Um die Gasbelastung für die Folgestufe gering zu halten, kann der Austrag aus dem Rohrreaktor in einer bevorzugten Ausführungsform einem Phasenscheider zugeführt und die dem Phasenscheider entnommene Flüssigphase dann der Folgestufe zugeführt werden.

Ein solcher Phasenscheider ist ein Behälter, in dem die Phasentrennung zwischen Gas- und Flüssigphase durch die Beruhigung der zweiphasigen, aus dem Gleichstromreaktor austretenden Strömung erreicht wird.

Der Phasenscheider kann isotherm oder bevorzugt beheizt ausgeführt werden, um das Ausfallen schwer löslicher Nebenprodukte zu verhindern. Die Beheizung kann beispielsweise über den Mantel oder über einen Kreislauf mit einem externen Wärmetauscher erfolgen. Bei Verwendung eines externen Wärmetauschers reicht eine normale Isolierung des Wärmetauschers.

Die Temperatur im Rohrreaktor und im gegebenenfalls vorhandenen Phasenscheider beträgt im allgemeinen zwischen 50 °C und 300 °C, bevorzugt zwischen 180 °C und 220 °C.

Der Druck in Stufe b) beträgt in der Regel zwischen 0,1 bar abs und 30 bar abs und bevorzugt zwischen 5 und 20 bar abs.

Das Überführen des Reaktionsaustrages aus der Stufe b) in die nachfolgende Stufe kann vorteilhaft über Druckhalteventile erfolgen, wobei der Druck in Stufe b) in der Regel mindestens 0,1 bar oberhalb des in Stufe c) herrschenden Druckes betragen sollte. Ist dies nicht der Fall, kann das Überführen z.B. mit Hilfe einer Pumpe oder barometrisch erfolgen.

Die Verweilzeit in Stufe b) ist so gewählt, daß der Umsatz, bezogen auf Aminogruppen im eingesetzten Diamin zu Urethangruppen, nach Verlassen des (Rohr)-Reaktors mindestens 95%, bevorzugt mindestens 98, besonders bevorzugt mindestens 99%, ganz besonders bevorzugt mindestens 99,5% und speziell mindestens 99,8 beträgt. Angestrebt werden Reaktionsbedingungen, die zu einem vollständigen Umsatz führen.

Üblicherweise beträgt die gesamte Verweilzeit in Stufe a) und b) zusammen weniger als 10 Stunden, bevorzugt weniger als 6 Stunden und besonders bevorzugt weniger als 4 Stunden.

Der Austrag der Reaktionsmischung aus (b) kann bei vollständigem Umsatz der Amingruppen zum Urethan direkt der Ammoniakabtrennung (c) zugeführt werden oder er wird zur Erzielung eines vollständigen Umsatzes einem weiteren Reaktor oder Reaktorsystem zugeführt. Als Reaktoren können weitere Rohrreaktoren, Mischreaktorkaskaden oder Kolonnen mit der notwendigen mittleren Verweilzeit zum Einsatz kommen.

Ist der Umsatz, bezogen auf Aminogruppen im eingesetzten Diamin zu Urethangruppen, nach Verlassen des Rohrreaktors noch nicht vollständig und beträgt beispielsweise weniger als 95%, so kann der Austrag nochmals nachreagiert werden.

Dazu kann das Reaktionsgemisch zur Vervollständigung des Umsatzes in einem weiteren Rohrreaktor oder aber auch in einem rückvermischten Reaktor nachreagieren gelassen werden, bevorzugt bis der Umsatz 98% oder mehr beträgt.

Unter rückvermischtem Reaktorsystem wird hier verstanden, dass die Bodensteinzahl des Reaktorsystems kleiner 5, bevorzugt kleiner 4 ist.

### c) Ammoniakabtrennung

Zur Abtrennung des Ammoniaks werden zweckmäßigerweise Kolonnen verwendet, bevorzugt wird der Ammoniak per Destillation abgetrennt. Dadurch gelingt eine gute Trennung zwischen dem Alkohol und Ammoniak. Üblicherweise erfolgt die Abtrennung in einem Druckbereich von 0,01 - 20 bar, vorzugsweise bei 0,04 - 15 bar. Die notwendigen Temperaturen richten sich nach dem verwendeten Alkohol bzw. dem Alkoholgemisch. Für n-Butanol liegt die Temperatur beispielsweise bei 60 - 150 °C, bevorzugt bei 80 bis 140 °C.

Es hat sich als vorteilhaft erwiesen, das entstehende Ammoniak sofort aus der Reaktionsmischung abzutrennen, so daß eine Belegung durch Ammoniumcarbaminat, welches in minimalen Mengen aus Ammoniak und Kohlendioxid durch Zersetzung von Harnstoff gebildet wird, vermieden werden kann.

Diese Destillationseinheit ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Bevorzugt werden Packungen verwendet.

Die Destillationskolonne weist bevorzugt 10 - 20 theoretische Trennböden auf.

### d) Abtrennung des überschüssigen Alkohols

Aus der erhaltenen ammoniakabgereicherten Reaktionsmischung werden dann Alkohol, das enthaltene Dialkylcarbonat oder in der Reaktionsmischung vorliegender Carbamidsäurealkylester, sofern solcher gebildet wurde, oder Mischungen aus mindestens zwei dieser Komponenten abgetrennt und zumindest teilweise in die erfindungsgemäße Stufe i) geführt.

Zur Abtrennung der Komponenten wird die Reaktionsmischung vorteilhafterweise vom Druckniveau der Reaktionsstufe (b) auf einen Druck im Bereich von 1 bis 500 mbar, vorzugsweise von 2 bis 100 mbar entspannt. Man erhält hierbei gasförmige Brüden, die die überwiegende Alkoholmenge sowie 1 bis 30 Gew.%, vorzugsweise 2 bis 10 Gew.% Dialkylcarbonat und/oder 1 bis 50 Gew.%, vorzugsweise 5 bis 15 Gew.% Carbamidsäurealkylester enthalten, und einen flüssigen Austrag, der im wesentlichen aus dem monomeren Diurethan besteht und gegebenenfalls Oligoharnstoffpolyurethanen und hochsiedende Oligomere enthält.

Die erhaltenen Brüden (d_{L}) werden in nachfolgenden zweckmäßigerweise destillativen Reinigungsstufen, vorzugsweise durch Rektifikation, getrennt und die hierbei isolierten Wertprodukte Alkohol, Carbonat und Carbamidsäurealkylester einzeln oder als Mischung, vorzugsweise in die Reaktionsstufe (a) zur Bildung der monomeren Urethane zurückgeführt bzw. zumindest teilweise in die erfindungsgemäße Stufe (i) geführt.

Es stellt eine bevorzugte Ausführungsform der vorliegenden Erfindung dar, daß zumindest ein Teil des abgetrennten carbonathaltigen Brüden (d_{L}) in die erfindungsgemäße Stufe (i) geführt wird. Darüberhinaus kann auch noch ein Teil des abgetrennten carbonathaltigen Brüden (d_{L}) in die Stufe (a) geführt werden.

Das Verhältnis des in die Stufe (a) geführten Stromes zu dem in die Stufe (i) geführten Strom beträgt von 0:100 bis 90:10, bevorzugt von 10:90 bis 90:10, besonders bevorzugt von 20:80 bis 80:20.

Als Alternative zu dieser beschriebenen Abtrennung kann es auch ein Möglichkeit darstellen, in einer ersten destillativen Abtrennung lediglich im wesentlichen den Alkohol als Leichtsieder aus dem Reaktionsgemisch abzutrennen und den Destillationssumpf, der neben Diurethan noch Dialkylcarbonat und optional Carbamidsäurealkylester enthält, einer weiteren Destillation zu unterwerfen, in dem Dialkylcarbonat und optional Carbamidsäurealkylester als Leichtsieder abgtrennt werden und das Diurethan im Destillationssumpf bleibt und in die nächste Stufe geleitet wird. Carbonat und Carbaminsäureester werden dann wie zuvor beschrieben zumindest teilweise in Stufe i) geleitet und der gegebenenfalls nicht in Stufe i) geleitete Rest in Stufe a) und/oder b) geführt.

Zur destillativen Abtrennung des Alkohols oder des Alkoholgemisches wird häufig ein sogenannter Flash eingesetzt. Dieser Apparat kann ein Behälter oder eine Kombination von Behälter und Kolonne vorzugsweise eine Kolonne sein, wobei im Kopf der Alkohol bzw. das Alkoholgemisch und im Sumpf das Urethan abgezogen werden kann. Im Kopf der Kolonne können neben dem Alkohol auch weitere leichter als das Urethan siedende Stoffe enthalten sein. Die Trennung erfolgt in einem Druckbereich von 0,001 bis 2 bar, vorzugsweise bei 0,02 - 0,5 bar.

### e) Urethanaufreinigung

Die in der Reaktionsstufe (d) nach Abtrennung der Brüden in der Regel als Sumpfaustrag erhaltene flüssige, die monomeren Diurethane und gegebenenfalls Oligoharnstoffpolyurethane und hochsiedende Oligomere enthaltende Reaktionsmischung (d) kann entweder vollständig in die Folgestufe geführt werden oder wird bevorzugt in zwei Teilströme geteilt, wobei das Gewichtsverhältnis der Teilmengen 5 bis 50:95 bis 50 Gew.-Teile, vorzugsweise 10 bis 30:90 bis 70 Gew.-Teile beträgt.

Die gleich große oder vorzugsweise größere Teilmenge wird destillativ getrennt mittels einer üblichen Destillationsanlage, vorzugsweise eines Dünnschichtverdampfers, bei einer Temperatur von 170 bis 240°C, vorzugsweise von 180 bis 230°C und unter einem Druck von 0,001 - 1 bar, vorzugsweise 0,002 - 0,01 bar, in ein Wertprodukt, das die Diurethane und die leichter siedenden Nebenprodukte enthält (e_{L}), und nicht destillierbare Nebenprodukte (e_{H}), die aus dem Herstellungsverfahren abgetrennt und üblicherweise als nicht verwertbarer Rückstand verworfen werden. Das Wertprodukt (Destillat) wird mit der gleich großen oder vorzugsweise größeren anderen Teilmenge vereinigt und die vereinigte Diurethane enthaltende Reaktionsmischung der thermischen Spaltung (f) zugeführt.

Durch diese Verfahrensmaßnahme in der Reaktionsstufe (e) wird der Anteil an nicht destillierbaren Nebenprodukten in der Reaktionsmischung, die sich bei den nacheinander ablaufenden Teilreaktionen bilden und durch die Rückführung verwertbarer Einsatzstoff im Reaktionskreislauf ständig anreichern würden, auf einen Gehalt von 3 bis 30 Gew.%, vorzugsweise 5 bis 20 Gew.% begrenzt und dadurch eine mit hoher Selektivität störungsfrei ablaufende Reaktion gewährleistet.

Als Destillationseinrichtungen können Dünnschichtverdampfer oder Kurzwegverdampfer zum Einsatz kommen. Das Urethan wird bei Drücken von 0,001 - 1 bar, vorzugsweise im Bereich von 0,002 - 0,01 bar destilliert. Das Destillat (e_{L}) wird der Spaltung (f) zugeführt.

Der hochsiederhaltige Sumpf (e_{H}) wird bevorzugt verworfen oder kann weniger bevorzugt teilweise der Reurethanisierung (h) zugeführt werden.

In einer bevorzugten Ausführungsform wird ein eventuell enthaltener restgehalt an Monomeren aus diesem hochsiederhaltige Sumpf abgetrennt. Dies kann beispielsweise erfolgen, wie in der Internationalen Patentanmeldung WO 2007/036479 beschrieben, die hiermit durch Bezugnahme Bestandteil der vorliegenden Offenbarungsei.

Bevorzugt wird der hochsiederhaltige Sumpf bei einer Temperatur zwischen 210 und 330 °C und einem Druck unter 300 hPa behandelt, so daß verwertbare Bestandteile, wie beispielsweise Monomere, ausgetrieben werden. Bevorzugt findet diese Behandlung statt in einem der folgenden Apparate:
a) Schaufeltrockner, bevorzugt ohne Kühlzone, bevorzugt mit Zwangsaustragsorganen,
b) Extruder mit Entgasungsmöglichkeit und
c) vertikale Dünnschichtprozessoren mit Zwangsaustragsorganen.

Besonders bevorzugt ist ein Schaufeltrockner.

### f) Urethanspaltung

Die in der Reaktionsstufe (e) erhaltene Diurethane enthaltende Reaktionsmischung wird in einer geeigneten Vorrichtung, bevorzugt lösungsmittelfrei in flüssiger Phase in Gegenwart von Katalysatoren bei Temperaturen von 200 bis 300°C, vorzugsweise 220 bis 280°C und unter vermindertem Druck von 0,01 - 0,6 bar, vorzugsweise im Bereich von 0,02 - 0,1 bar kontinuierlich thermisch gespalten. Der Umsatz des Urethanes in der Vorrichtung zur thermischen Spaltung kann in Abhängigkeit vom verwendeten Urethan weitgehend frei gewählt werden und liegt zweckmäßigerweise in einem Bereich von 10 bis 98 Gew.%, vorzugsweise 40 bis 90 Gew.% der zugeführten Urethanmenge.

Der ungespaltene Anteil der Reaktionsmischung, der nicht umgesetzte Urethane, Oligoharnstoff-polyurethane, hochsiedende Oligomere und andere wieder verwertbare und unverwertbare Nebenprodukte enthält, wird abgetrennt, kontinuierlich aus der Spaltvorrichtung ausgeschleust (f_{H}) und direkt oder gegebenenfalls nach Umsetzung mit Alkohol in der Reurethanisierung (h) in die Reaktionsstufe (a) und/oder (b) zurückgeführt.

Als Katalysatoren zur chemischen Spaltung der Urethane finden z.B. die vorgenannten, die Urethanbildung katalysierenden anorganischen und organischen Verbindungen Verwendung.

Besonders bewährt und daher vorzugsweise verwendet werden Dibutylzinndilaurat, Eisen-(III)-acetylacetonat, Kobalt-(II)-acetylacetonat, Zinkacetylacetonat, Zirkon tetra-n-butanolat und Zinn-(II)-dioctoat.
Als Spaltvorrichtungen eignen sich beispielsweise zylinderförmige Spaltreaktoren, wie z.B. Röhrenöfen oder vorzugsweise Verdampfer, beispielsweise Dünnschicht- oder Bulkverdampfer, wie z.B. Robertverdampfer, Herbertverdampfer, caddle-typ-Verdampfer, Plattenspalter, Heizkerzenverdampfer, vorzugsweise Plattenspalter.

Die Trennung der Spaltprodukte erfolgt in einer Kolonne, bei der üblicherweise das Isocyanat in der Seite (f_{M}) und der Alkohol (f_{L}) am Kopf abgezogen werden.

Nach einer bevorzugten Ausführungsform wird die bei der destillativen Reinigung des rohen Isocyanats (f) anfallende Kopffraktion in die Reaktionsstufe (a) zurückgeführt, die Seitenfraktion, die aus im wesentlichen reinem Isocyanat besteht, wird einem Behälter zur Lagerung und die Sumpffraktion in die Reaktionsstufe (a) oder (d) oder (a) und (d) übernommen.

### g) Isocyanatreinigung

Das Rohisocyanatgemisch wird in einer sich anschließenden Destillation von Rekombinationsprodukten, Nebenprodukten und sofern vorhanden dem Lösungsmittel befreit. Die Nebenprodukte werden vorzugsweise in die thermische Spaltung zurückgeführt. Ein Teil kann auch ausgeschleust werden.

Die bei der thermischen Spaltung gebildeten Spaltprodukte, die sich vor allem aus Alkohol Diisocyanat, und partiell gespaltenen Urethanen zusammensetzen, werden danach vorteilhafterweise mit Hilfe einer oder mehrerer Destillationskolonnen, vorzugsweise durch Rektifikation bei Temperaturen von 100 bis 240°C, vorzugsweise 120 bis 200°C und einem Druck von 1 bis 200 mbar, vorzugsweise 5 bis 50 mbar, in Leichtsieder und besonders Alkohol (g_{L}) und eine rohe Isocyanatmischung (g_{M}) mit einem Isocyanatgehalt von 85 bis 99 Gew.%, vorzugsweise von 95 bis 99 Gew.% getrennt. Die bei der destillativen Trennung anfallenden höhersiedenden Nebenprodukte (g_{H}) und insbesondere die ungespaltenen und partiell gespaltenen Urethane werden vorzugsweise in die Spaltvorrichtung (f) und/oder Reurethanisierung (h) geführt.

Mit dem Index "L" werden hier leichtsiedende Ströme der einzelnen Stufen gekennzeichnet, mit dem Index "H" hochsiedende und mit "M" mittelsiedende.

Die vorzugsweise durch Rektifikation erhaltene rohe Isocyanatmischung (g_{M}) wird durch Destillation bei einer Temperatur von 100 bis 180°C und unter einem Druck von 1 bis 50 mbar gereinigt, wobei die einzelnen Fraktionen zurückgeführt oder als Reinprodukt isoliert werden. Wie bereits ausgeführt wurde, wird bei der bevorzugt angewandten Reindestillation die Kopffraktion, die vorzugsweise aus Diisocyanat besteht, gegebenenfalls nach Umsetzung der freien Isocyanatgruppen mit Alkohol in die Reaktionsstufe (a) und/oder (b) zurückgeführt, die Seitenfraktion, die aus reinem Diisocyanat, vorzugsweise mit einer Reinheit von mindestens 98 Gew.%, insbesondere über 99,8 Gew.% besteht, wird abgeleitet und der Lagerung zugeführt und die Sumpffraktion, die als wesentliche Komponenten die partiell gespaltenen Urethane und Isocyanate enthält, wird vorzugsweise in die Spaltvorrichtung zur thermischen Spaltung zurückgeführt.

Nach anderen Verfahrensvarianten kann die Sumpffraktion (g_{H}) jedoch auch in die Destillationskolonne (d) zur Trennung von rohem Diisocyanat und Alkohol oder in die Reaktionsstufe (a) und/oder (b), die Urethanbildung, zurückgeführt werden. Möglich ist auch eine Teilung der Sumpffraktion in 2 oder 3 Produktströme, wobei diese vorzugsweise in der Urethanbildung (a) und/oder die Spaltvorrichtung (f) sowie gegebenenfalls in die Destillationskolonne (g) und/oder in die Reurethanisierung (h) zurückgeführt werden.

### h) Reurethanisierung

Die Umsetzung des Reaktionsaustrages (f_{H}) aus f) und/oder Destillationsrückstände (g_{H}) aus (g) werden vorzugsweise erneut dem Prozess zugeführt. Dabei werden mit Alkohol die in diesem Gemisch enthaltenen Isocyanatgruppen und/oder Allophanate und/oder Harnstoffe oder sonstige reaktive Bestandteile zu Urethanen umgewandelt. Es besteht die Möglichkeit, diese Reaktionen in separaten Reaktoren wie z. B. Mischreaktoren oder Strömungsrohren oder auch in (b) durchzuführen. Für die Alkoholyse der Rückstände sind Temperaturen von 100 - 250 °C, vorzugsweise 150 - 220 °C erforderlich. Die mittleren Verweilzeiten liegen dabei im Bereich von wenigen Minuten bis Stunden. In der Regel wird die Reaktion bevorzugt einphasig in der Flüssigphase durchgeführt. Der Druck während der Reaktion spielt dabei keine besonders Rolle, außer daß er ausreichend sein sollte, um das Reaktionsgemisch in der Flüssigphase zu halten.

Dazu können beispielsweise die Ströme (f_{H}) und/oder (g_{H}) sowie gegebenenfalls ein Teil des Stromes (e_{H}) mit Alkohol zusammengeführt werden, wobei das Molverhältnis von NCO-Gruppen bzw. deren Äquivalenten, also beispielsweise Urethangruppen, zu Hydroxygruppen bis zu 1:100, bevorzugt bis zu 1:20, besonders bevorzugt bis zu 1:10 beträgt.

Der Alkohol kann dabei beispielsweise der leichtsiedende Strom (d_{L}) aus der Stufe (d) und/oder der alkohohaltige Strom (f_{L}) aus der Urethanspaltung (f) und/oder auch frischer Alkohol sein.

Die Reaktionsmischung wird in Gegenwart oder Abwesenheit von Katalysatoren innerhalb von 1 bis 150 min, bevorzugt 3 bis 60 min bei Temperatur von 20 bis 200 °C, bevorzugt 50 bis 170 °C bei einem Druck von 0,5 bis 20 bar, bevorzugt 1 bis 15 bar umgesetzt.

Wenn ein Katalysator eingesetzt werden sollte, so handelt es sich bevorzugt um den gleichen Katalysator wie in der Stufe der Urethanbildung (b).

Die Umsetzung kann in einer kontinuierlichen Kesselkaskade oder in einem Rohrreaktor durchgeführt werden.
Als Katalysatoren kommen grundsätzlich alle Verbindungen in Frage, die die Reaktion von NCO- mit OH-Gruppen fördern. Beispielsweise seien genannt Zinnoctoat, Dibutylzinndilaurat, Zinnchlorid, Zinkdichlorid, Zinn-(II)-dioctoat und Triethylamin.

### i) Umsetzung des Carbonats

Erfindungsgemäß wird mindestens ein carbonathaltiger Strom mit Ammoniak unter Umwandlung zumindest eines Teils des Carbonats in den entsprechenden Carbaminsäureester umgesetzt.

Der carbonathaltige Strom kann neben Carbonat noch Alkohol in Mengen bis zu 5 Gew% sowie Carbaminsäureester in Mengen von bis zu 25 Gew% enthalten. Ferner kann Ammoniak in Mengen bis zu ca. 0,5 Gew% enthalten sein. Weitere Komponenten können in ebenfalls geringen Mengen enthalten sein, wenn diese in dem Intervall der Siedepunkte von Carbamat und Carbaminsäureester eine Flüchtigkeit aufweisen.

In der Stufe (i) wird Ammoniak mit Carbonat im Molverhältnis 1:1 bis 100:1, bevorzugt 1,5:1 bis 80:1 und besonders bevorzugt 2:1 bis 50:1 umgesetzt.

Die Temperatur in der Umsetzung sollte in der Regel 50 bis 250 °C betragen, bevorzugt 50 bis 200 °C und besonders bevorzugt 60 bis 180 °C.

Die Dauer der Umsetzung beträgt in der Regel 1 min bis 6 Stunden, bevorzugt 5 min bis 4 Stunden und besonders bevorzugt 10 min bis 2 Stunden.

Der Druck in der Umsetzung sollte 1 bar (abs) bis 50 bar, bevorzugt 2 bis 40 bar, besonders bevorzugt 3 bis 30 bar und ganz besonders bevorzugt 5 bis 20 bar betragen.

Optional kann mindestens ein Katalysator wie unter (a) beschrieben in der Reaktion anwesend sein, bevorzugt ist mindestens ein Katalysator anwesend.

Die Stufe (i) kann diskontinuierlich oder kontinuierlich, bevorzugt kontinuierlich durchgeführt werden.

Bevorzugt führt man die Umsetzung unter ständiger Abtrennung des gebildeten Akohols durch, beispielsweise in einer Rührkesselkaskade mit aufgesetzten Destillationskolonnen oder in einer Reaktionsdestillation.

Besonders bevorzugt findet die Stufe (i) in einem Rohrreaktor statt, wobei man das Reaktionsgemisch im Abschluß zur Abtrennung des Ammoniak entspannt:
In einer weiteren Ausführungsform wird das aus der Stufe d) erhaltene Carbonat destillativ von den anderen Leicht- und Mittelsiedern getrennt, in einem Verdampfer bei Temperaturen von 100 - 250 °C verdampft und in der Gasphase in einem Rohrreaktor mit Ammoniakgas umgesetzt. Der Druck in diesem Verweilzeitreaktor kann von 20 mbar -10 bar, bevorzugt 100 mbar bis 9 bar, besonders bevorzugt 1 bis 8 bar, ganz besonders bevorzugt 1,1 bis 7 bar und insbesondere 1,2 bis 6 bar abs eingestellt werden. In einer Ausführungsform des Gasphasenreaktors wird die Reaktion an einer katalytisch aktiven Oberfläche beschleunigt.

Wird die Gasphasenreaktion bei erhöhtem Druck durchgeführt, so stellt es eine bevorzugt Ausführungsform dar, das Reaktionsgemisch auf normalen Druck zu entspannen und die flüssigen Reaktionsprodukte zu kondensieren. Der überschüssige Ammoniak kann anschließend komprimiert und wieder in die Reaktion zurückgeführt oder thermisch verwertet werden, beispielsweise wie beschrieben in WO 2008/025659.

Dies kann in einer gesonderten Abtrennung erfolgen, aber auch beispielsweise in der oben beschriebenen Stufe c) erfolgen.

Das Reaktionsgemisch der Stufe (i), in dem zumindest ein Teil des Carbonat in Carbaminsäureester umgewandelt ist, kann dann in Stufe a) und/oder b) geführt werden.

Bevorzugt wird das Reaktionsgemisch der Stufe (i), besonders bevorzugt nach Abtrennung des Ammoniak wie beschrieben, jedoch in die Stufe (d) geführt und dort destillativ in einen überwiegend carbonathaltigen Strom und einen Strom, der überwiegend Carbaminsäureester enthält. Der carbonathaltge Strom wird von dort wieder in Stufe i) geführt und der Carbaminsäureester enthaltende Strom wird in die Stufe a) und/oder b) geleitet.

Mit dem erfindungsgemäßen mehrstufigen Verfahren zur kontinuierlichen Herstellung von Diisocyanat unter Rückführung und Ausschleusung der Nebenprodukte kann das als Nebenprodukt entstehende Carbonat gut verwertet werden.

Das so hergestellte Diisocyanat eignet sich vorzüglich zur Herstellung von Urethan-, Isocyanurat-, Amid- und/oder Harnstoffgruppen enthaltenden Kunststoffen nach dem Polyisocyanat-Polyadditionsverfahren. Sie finden ferner Verwendung zur Herstellung von mit Urethan-, Biuret- und/oder Isocyanuratgruppen modifizierten Polyisocyanatmischungen. Derartige Polyisocyanatmischungen aus aliphatischen oder cycloaliphatischen Diisocyanaten werden insbesondere zur Herstellung von lichtbeständigen Polyurethanlacken und -überzügen verwendet.

Die folgenden Beispiele sollen die Erfindung erläutern, aber nicht auf diese Beispiele einschränken.

### Beispiel

### Beispiel 1 - Reaktion in der flüssigen Phase

In einem 1,5 Liter Reaktor mit Rührer werden 600 g eines Gemisches aus 77% Di-n-butylcarbonat, 11 % O-Butylcarbamat, 2% n-Butanol und weiteren Nebenverbindungen vorgelegt. Zusätzlich werden 500 ppm Katalysator in das Gemisch gegeben. Als Katalysator wurde Zirkontetrabutylat verwendet. Der Druck von 5 bar wird während der sich anschließenden Reaktion mit einer angeschlossenen Ammoniakflasche konstant gehalten. Der Reaktor wird schnell auf 210 °C aufgeheizt und vier Stunden gerührt. Danach wird abgekühlt und entspannt. Das Reaktionsgemisch enthielt 24,6% Di-n-butylcarbonat,38% O-Butylcarbamat und 26,2% n-Butanol. Der Carbonatumsatz erreichte ca. 68%.

### Vergleichsbeispiel 1

In einem Vergleichsbeispiel analog Beispiel 1 wurde die Reaktion nur mit Stickstoff statt mit Ammoniak durchgeführt. Es konnte kein Umsatz beobachtet werden.

### Beispiel 2 - Gasphasenumsetzung

In einem umgebauten Gaschromatographen, gekoppelt mit einem Massenspektrometer, mit einer 4 Meter langen Säule von 0,1 mm Durchmesser konnten bei einer Injektor-, Ofen- und Detektortemperatur von 200 °C bereits ein Umsatz von 52,8% des eingespritzten Di-n-Butylcarbonates analysiert werden. Als Träger- bzw. Reaktionsgas wurde eine Mischung von 50% Ammoniak mit Helium verwendet. Die Auswertung erfolgte über die charakteristischen Bruchstücke der Verbindungen. Eine Kalibrierung mit einem Tracer war notwendig.

## Patentansprüche

1. Verfahren zur Herstellung von Diisocyanaten durch Umsetzung der korrespondierenden Diamine mit Harnstoff und mindestens einem Alkohol zum korrespondierenden Urethan (Urethanisierung) unter Bildung des Carbonats des eingesetzten Alkohols und Spaltung des so erhaltenen Urethans zum Isocyanat (Urethanspaltung), **dadurch gekennzeichnet, daß** man zumindest einen Teil des gebildeten Carbonats aus dem Reaktionsgemisch abtrennt, außerhalb der Urethanisierung mit Ammoniak umsetzt und das so gebildete Reaktionsgemisch in die Urethanisierung führt.

2. Verfahren gemäß Anspruch 1, **dadurch gekennzeichnet, daß** man den Austrag aus der Urethanisierung im wesentlichen von Ammoniak befreit, man aus dem so erhaltenen Gemisch Alkohol, Carbonat sowie gegebenenfalls Carbaminsäureester abtrennt und das so erhaltene Carbonat zumindest teilweise mit Ammoniak umsetzt.

3. Verfahren gemäß Anspruch 2, **dadurch gekennzeichnet, daß** die Umsetzung von Ammoniak mit Carbonat im Molverhältnis 1:1 bis 100:1 erfolgt.

4. Verfahren gemäß einem der vorstehenden Ansprüche, **dadurch gekennzeichnet, daß** die Umsetzung von Ammoniak mit Carbonat bei 50 bis 250 °C erfolgt

5. Verfahren gemäß einem der vorstehenden Ansprüche, **dadurch gekennzeichnet, daß** die Umsetzung von Ammoniak mit Carbonat in Gegenwart mindestens eines Katalysators erfolgt.

6. Verfahren gemäß einem der vorstehenden Ansprüche, **dadurch gekennzeichnet, daß** die Umsetzung von Ammoniak mit Carbonat unter ständiger Abtrennung des gebildeten Akohols erfolgt.

7. Verfahren gemäß einem der vorstehenden Ansprüche, **dadurch gekennzeichnet, daß** das Amin ausgewählt ist aus der Gruppe bestehend aus Dicyclohexylmethylendiamin (H12MDA), Hexandiamin-1,6 und 3-Aminomethyl-3,5,5-trimethylcyclohexylamin.

8. Verfahren gemäß einem der vorstehenden Ansprüche, **dadurch gekennzeichnet, daß** der Alkohol ausgewählt ist aus der Gruppe bestehend aus Methanol, n-Butanol und iso-Butanol.

## Claims

1. A process for preparing diisocyanates by reaction of the corresponding diamines with urea and at least one alcohol to form the corresponding urethane (urethanization) with formation of the carbonate of the alcohol used and dissociation of the urethane obtained in this way to give the isocyanate (urethane dissociation), wherein at least part of the carbonate formed is separated off from the reaction mixture, reacted with ammonia outside the urethanization and the reaction mixture formed in this way is introduced into the urethanization.

2. The process according to claim 1, wherein the output from the urethanization is substantially freed of ammonia, and alcohol, carbonate and, if present, carbamic ester are separated off from the resulting mixture and the carbonate obtained in this way is at least partly reacted with ammonia.

3. The process according to claim 2, wherein the reaction of ammonia with carbonate is carried out at a molar ratio of from 1:1 to 100:1.

4. The process according to any of the preceding claims, wherein the reaction of ammonia with carbonate is carried out at from 50 to 250°C.

5. The process according to any of the preceding claims, wherein the reaction of ammonia with carbonate is carried out in the presence of at least one catalyst.

6. The process according to any of the preceding claims, wherein the reaction of ammonia with carbonate is carried out with continual removal of the alcohol formed.

7. The process according to any of the preceding claims, wherein the amine is selected from the group consisting of dicyclohexylmethylenediamine (H12MDA), 1,6-hexanediamine and 3-aminomethyl-3,5,5-trimethylcyclohexylamine.

8. The process according to any of the preceding claims, wherein the alcohol is selected from the group consisting of methanol, n-butanol and isobutanol.

## Revendications

1. Procédé pour la préparation de diisocyanates par transformation des diamines correspondantes avec de l'urée et au moins un alcool en uréthane correspondant (uréthanisation) avec formation du carbonate de l'alcool utilisé et par dissociation de l'uréthane ainsi obtenu en isocyanate (dissociation d'uréthane), **caractérisé en ce qu'**on sépare au moins une partie du carbonate formé du mélange réactionnel, on la transforme en dehors de l'uréthanisation avec de l'ammoniac et on introduit le mélange réactionnel ainsi formé dans l'uréthanisation.

2. Procédé selon la revendication 1, **caractérisé en ce qu'**on libère sensiblement le produit sorti de l'uréthanisation de l'ammoniac, on sépare du mélange ainsi obtenu l'alcool, le carbonate ainsi que le cas échéant l'ester de l'acide carbamique et on transforme le carbonate ainsi obtenu au moins partiellement avec de l'ammoniac.

3. Procédé selon la revendication 2, **caractérisé en ce que** la transformation de l'ammoniac avec le carbonate a lieu dans un rapport molaire de 1:1 à 100:1.

4. Procédé selon l'une quelconque des revendications précédentes, **caractérisé en ce que** la transformation de l'ammoniac avec le carbonate a lieu à 50 jusqu'à 250°C.

5. Procédé selon l'une quelconque des revendications précédentes, **caractérisé en ce que** la transformation de l'ammoniac avec le carbonate a lieu en présence d'au moins un catalyseur.

6. Procédé selon l'une quelconque des revendications précédentes, **caractérisé en ce que** la transformation de l'ammoniac avec le carbonate a lieu sous une séparation continue de l'alcool formé.

7. Procédé selon l'une quelconque des revendications précédentes, **caractérisé en ce que** l'amine est choisie dans le groupe constitué par la dicyclohexylméthylènediamine (H12MDA), l'hexanediamine-1,6 et la 3-aminométhyl-3,5,5-triméthylcyclohexylamine.

8. Procédé selon l'une quelconque des revendications précédentes, **caractérisé en ce que** l'alcool est choisi dans le groupe constitué par le méthanol, le n-butanol et l'iso-butanol.
